# EUROPÄISCHE PATENTSCHRIFT

(11) **EP 3 363 358 B1**
(45) Veröffentlichungstag und Bekanntmachung des Hinweises auf die Patenterteilung: **21.10.2020**
(21) Anmeldenummer: 18156280.2
(22) Anmeldetag: 12.02.2018
(51) Int. Cl.: A61B 5/06, A61B 34/35, A61B 34/20, A61B 1/00, A61B 1/04, A61B 1/07, A61B 5/00, G01B 11/00, G06T 7/00, A61B 34/30

(54) **VORRICHTUNG ZUM FESTLEGEN UND WIEDERAUFFINDEN EINES BEZUGSPUNKTS WÄHREND EINES CHIRURGISCHEN EINGRIFFS**
DEVICE FOR DETERMINING AND RETRIEVING A REFERENCE POINT DURING A SURGICAL OPERATION
DISPOSITIF DE DÉTERMINATION ET RECOUVREMENT D'UN POINT DE RÉFÉRENCE LORS D'UNE INTERVENTION CHIRURGICALE

(30) Priorität: 16.02.2017 DE 102017103198
(43) Veröffentlichungstag der Anmeldung: 22.08.2018
(73) Patentinhaber: avateramedical GmbH, 07745 Jena (DE)
(72) Erfinder: Seeber, Marcel, 07745 Jena (DE)
(74) Vertreter: Schaumburg und Partner Patentanwälte mbB

(56) Entgegenhaltungen:
- EP-A1- 2 411 966
- EP-A1- 2 942 029
- WO-A1-2016/018648
- DE-A1-102015 109 371
- DE-C1- 10 033 723
- US-A- 5 749 362
- US-A1- 2003 018 235

## Beschreibung

Die Erfindung betrifft eine Vorrichtung zum Festlegen und Wiederauffinden eines Bezugspunkts während eines chirurgischen Eingriffs. Die Vorrichtung umfasst ein Endoskop, das mehrere Bilder nacheinander als Bildfolge erfasst und den Bildern entsprechende Bilddaten erzeugt. Mindestens eine Steuereinheit verarbeitet die Bilddaten und gibt den Bilddaten entsprechende Bilder auf mindestens einer Anzeigeeinheit aus. Durch eine Bedieneingabe über eine Benutzerschnittstelle wird ein Bezugspunkt festgelegt, der im weiteren Verlauf des chirurgischen Eingriffs wieder auffindbar sein soll.

Die Vorrichtung wird insbesondere in einem System zur robotergestützten Chirurgie, insbesondere zu einer telerobotergestützten Prozedur, eingesetzt. Das System hat mindestens einen mit dem Endoskop verbundenen Manipulatorarm und/oder einen mit einem chirurgischen Instrument zur Gewebemanipulation verbundenen Manipulatorarm. Solche Systeme werden auch als Manipulatoren oder Telemanipulatoren oder OP-Roboter bezeichnet.

In den Dokumenten DE 10 2015 109 368 und DE 10 2015 109 371 sind Vorrichtungen und Verfahren zur telerobotergestützten Chirurgie offenbart.

Bei minimalinvasiven ausgeführten chirurgischen Eingriffen, insbesondere bei der Laparoskopie, werden die für Gewebemanipulationen beim chirurgischen Eingriff erforderlichen chirurgischen Instrumente durch sogenannte Trokare in den zu operierenden Patienten eingeführt. Zusätzlich zu den zur Gewebemanipulation einzuführenden chirurgischen Instrumenten wird ein Endoskop durch einen weiteren Trokar zur Aufnahme von Bildern des Operationssitus während des chirurgischen Eingriffs in den Körper des Patienten eingeführt. Beim chirurgischen Eingriff nutzt der Chirurg das auf einer Anzeigeeinheit dargestellte Livebild des Operationssitus, um sich zu orientieren, erforderliche Gewebemanipulationen festlegen und in Echtzeit ausführen zu können sowie für den Therapieverlauf relevante Entscheidungen treffen zu können. Dazu hat der Chirurg zumindest einen Teil der Endeffektoren der Instrumente, mit denen er das Gewebe manipuliert, im Sichtfeld des Endoskops. Diese Arbeitsweise ist sowohl bei manuell ausgeführten chirurgischen Eingriffen mit manuell betätigbaren chirurgischen Instrumenten als auch bei chirurgischen Eingriffen, die mithilfe eines Telemanipulators bei einer robotergestützten Chirurgie durchgeführt werden, gleich. Bei dem Einsatz eines Telemanipulators werden die Position und die Orientierung der chirurgischen Instrumente sowie die Position und Orientierung des Endoskops durch Manipulatorarme des Telemanipulators geführt. Der Chirurg steuert die Manipulatorarme und die chirurgischen Instrumente durch haptische Eingabegeräte fern.

Im Verlauf eines chirurgischen Eingriffs kann es für den Chirurgen erforderlich sein, dass er das Endoskop auf bestimmte anatomische Merkmale oder Strukturen ausrichtet, um insbesondere eine gute Sicht auf zu manipulierendes Gewebe oder auf plötzlich auftretende Blutungen auszurichten. Nur so ist es dem Chirurgen möglich, die relevanten Merkmale oder Strukturen visuell zu begutachten und Entscheidungen für den weiteren Therapieverlauf zu treffen. Dabei ist es auch möglich, dass ein chirurgisches Instrument oder mehrere chirurgische Instrumente aus dem Sichtfeld des Endoskops verschwinden, so dass der Chirurg keine visuelle Kontrolle mehr über die Instrumente hat. Für eine Gewebemanipulation ist jedoch eine Sichtkontrolle erforderlich, so dass der Chirurg das Endoskop vor einer Gewebemanipulation wieder so auszurichten hat, dass er die Instrumente im Sichtfeld hat, die er zur Gewebemanipulation nutzen will. Grundsätzlich sollte zur Patientensicherheit ein Bewegen der Instrumente ohne Sichtkontrolle vermieden werden.

Aus dem Dokument EP 2411966 B1 ist ein System zur visuellen Führung zum Positionieren einer Spitze einer endoskopischen Einrichtung zu einer oder mehreren Landmarken und zur Unterstützung des Bedieners bei einer endoskopischen Navigation bekannt.

Aus dem Dokument EP 2046538 B1 ist ein System bekannt, bei dem ein Identifikationsindikator und die Position eines chirurgischen Instruments in einem Randbereich eines Computeranzeigebildschirms angezeigt werden.

Aus dem Dokument US 2003/0018235 A1 ist ein Assistenzsystem zur besseren Visualisierung der inneren anatomischen Struktur eines Patienten bei einem endoskopischen Videosystem bekannt. Bei diesem System werden nicht weiter konkretisierte Trackingsysteme mit Komponenten des Systems sowie mit Personen verbunden. So erhält ein Endoskop ein erstes Trackingsystem, der Chirurg ein zweites Trackingsystem und eine Anzeigeeinheit ein drittes Trackingsystem. Sowohl vom gescannten Patienten als auch vom einzelnen anatomischen Objekt werden 3D-Modelle erzeugt, die dann zur besseren Information des Chirurgen genutzt werden können.

Aufgabe der Erfindung ist es, eine Vorrichtung zum Festlegen und Wiederauffinden eines Bezugspunkts während eines chirurgischen Eingriffs anzugeben, durch das einem Chirurgen während eines chirurgischen Eingriffs das Wiederauffinden des Bezugspunkts erleichtert wird.

Diese Aufgabe wird sowohl durch eine Vorrichtung zum Festlegen und Wiederauffinden eines Bezugspunkts während eines chirurgischen Eingriffs mit den Merkmalen des Patentanspruchs 1 gelöst. Vorteilhafte Weiterbildungen sind für beide Vorrichtungen in den abhängigen Ansprüchen angegeben.

Durch den ermittelten Abstandsvektor zwischen der ersten Position und der zweiten Position kann bei der Vorrichtung nach Anspruch 1 der festgelegte Bezugspunkt mithilfe der auf dem Abstandsvektor basierenden Information einfach wiedergefunden werden, wobei ein Chirurg das Endoskop insbesondere so bewegen kann, dass der Bezugspunkt wieder in den Sichtbereich des Endoskops gelangt.

Allgemein hat die Erfindung den Zweck, eine Information zu erzeugen, die dazu verwendet werden kann, dem Chirurgen durch die Ausgabe visueller, haptischer oder akustischer Signale ein Ausrichten des Endoskops derart zu ermöglichen, dass die Instrumente und/oder ein anderer Bezugspunkt wieder im Sichtfeld des Endoskops sind. Bei der Ausgabe visueller Informationen in Verbindung mit einer 3D-Anzeige, wie einem stereoskopischen Display, kann eine Richtungsinformation insbesondere als 3D-Vektor angezeigt werden. Ferner hat die Erfindung den Zweck, dass ein Chirurg während eines chirurgischen Eingriffs einen Bezugspunkt bzw. eine virtuelle Landmark, im Operationssitus definieren kann, wobei eine Information erzeugt wird, welche dem Chirurgen das Wiederauffinden dieses Bezugspunkts durch die Ausgabe visueller, haptischer oder akustischer Signale ermöglicht. Hierdurch ist es dem Chirurgen insbesondere möglich, das Endoskop so auszurichten, dass der Bezugspunkt im Sichtfeld des Endoskops erscheint.

Die erste und zweite Position nach Anspruch 1 können insbesondere in einem dreidimensionalen Patientenkoordinatensystem oder einem dreidimensionalen Instrumentenkoordinatensystem ermittelt werden.

Vorteilhaft ist es, wenn die Steuereinheit einen Bereich um einen festgelegten Bezugspunkt, d.h. um den festgelegten ersten Bezugspunkt oder einen festgelegten weiteren Bezugspunkt, mithilfe einer Markierung markiert oder dass die Steuereinheit einen Bereich um einen festgelegten Bezugspunkt des chirurgischen Instruments mithilfe einer Markierung markiert. Dieser Bezugspunkt des chirurgischen Instruments, kann die Instrumentenspitze, insbesondere die Spitze des Endeffektors oder eine Schnittstelle zwischen Schaft und Endeffektor, sein. Der Bezugspunkt nach Anspruch 1 bzw. der Bezugspunkt am chirurgischen Instrument dient somit als Orientierungspunkt. Aufgrund der bekannten Kinematikstruktur bzw. der kinematischen Kette können die genauen Koordinaten der Punkte, insbesondere der Orientierungspunkte und der Bezugspunkte bestimmt werden. Die auf dem Abstandsvektor basierende ausgegebene Information dient als Orientierungshilfe beim Bewegen eines Endoskops, um beispielsweise den Bezugspunkt des chirurgischen Instruments oder ein zuvor festgelegter Bezugspunkt in den Sichtbereich des Endoskops zu bringen.

Eine Markierung des Bereichs um den festgelegten Bezugspunkt erfolgt vorzugsweise dadurch, dass ein Organ am Bezugspunkt oder in unmittelbarer Nähe des Bezugspunkts befindliches Organ oder eine am Bezugspunkt oder in unmittelbarer Nähe des Bezugspunkts befindliche Gewebestruktur, insbesondere mithilfe eines Mustererkennungsverfahrens, detektiert und dieses Organ bzw. diese Gewebestruktur mit einer Markierung versehen wird. Insbesondere können die Gewebestruktur bzw. das Organ zur Markierung eingefärbt dargestellt werden. Somit ist es einfach möglich, mithilfe der festgelegten Bezugspunkte, die als anatomische Landmarken dienen, mittels eines nachfolgend vom Endoskop aufgenommenen Bildes wiederzuerkennen, indem eine Extraktion von geeigneten Objektmerkmalen zur Klassifizierung um des Organs oder der Gewebestruktur oder des Bezugspunkts erfolgt.

Besonders vorteilhaft ist es, wenn die Steuereinheit einen Bildausschnitt der Umgebung der ermittelten ersten Position beim Ermitteln der ersten Position erfasst und zum Wiederauffinden der ersten Position in einem nach dem Ermitteln der ersten Position erfassten weiteren Bild mit mindestens einem Bildausschnitt des weiteren Bildes vergleicht. Hierzu kann die Steuereinheit insbesondere Verfahren der Mustererkennung von Gewebestrukturen und zur Mustererkennung von Organen ausführen. Alternativ oder zusätzlich kann ein automatisches Auffinden der ersten Position mithilfe der Steuereinheit durch Ausführen eines Verfahrens zur Korrelation zwischen den abgespeicherten Objektmerkmalen eines an der ersten Position detektierten Objekts und den aus einem jeweils aktuell aufgenommenen Bild extrahierten Objektmerkmalen erfolgen. Das automatische Auffinden durch einen Bildvergleich erfolgt insbesondere unter Berücksichtigung von Rotation, Translation, Skalierung und Verformung, wobei beispielsweise ein sogenanntes Warping-Verfahren eingesetzt wird. Hierdurch sind ein Wiederauffinden und vorzugsweise auch ein Markieren der ersten Position in einem weiteren Bild einfach möglich.

Ferner ist es vorteilhaft, wenn die Steuereinheit die erste Position ausgehend von einem mithilfe des Endoskops erfassten ersten Bildes oder ausgehend von der Position des Endoskops zum ersten Zeitpunkt oder ausgehend von der Position des chirurgischen Instrumentes ermittelt, und wenn die Steuereinheit die zweite Position ausgehend von einem nach dem ersten Bild mithilfe des Endoskops erfassten zweiten Bildes oder ausgehend von der Position des Endoskops zum zweiten Zeitpunkt ermittelt. Hierdurch muss die Position des Punkts nicht aufwendig ermittelt werden, sondern kann einfach aus der Position des Endoskops bzw. der Position des chirurgischen Instruments bestimmt werden.

Ferner ist es vorteilhaft, wenn die Steuereinheit die Positionen jeweils mithilfe der kinematischen Kette eines mit dem Endoskop verbundenen Manipulatorarms eines Manipulators bzw. eines chirurgischen Robotersystems oder mithilfe der kinematischen Kette eines mit dem chirurgischen Instrument verbundenen Manipulatorarms des Manipulators oder des chirurgischen Robotersystems ermittelt. Hierdurch ist eine einfache Positionsbestimmung in der Position des ersten Punkts, des zweiten Punkts und des chirurgischen Instruments möglich. Insbesondere kann die Änderung der Positionen infolge einer Bewegung des jeweiligen Manipulatorarms einfach nachvollzogen und die relevante Position dann einfach über die kinematische Kette bzw. die Änderung der Lage des Endoskops bzw. des chirurgischen Instruments bestimmt werden.

Ferner ist es vorteilhaft, wenn das Endoskop zum ersten Zeitpunkt ein erstes Bild erfasst und wenn das Endoskop zum zweiten Zeitpunkt ein zweites Bild erfasst bzw. wenn das Endoskop beim Ermitteln der ersten Position ein erstes Bild erfasst und wenn das Endoskop beim Ermitteln der zweiten Position ein zweites Bild erfasst. Hierdurch können die Bildausschnitte beim Ermitteln der Positionen mit Bildausschnitten späterer Bilder verglichen werden. Ferner können die erfassten Bilder gespeichert und auf Anforderung nochmals angezeigt werden, damit die Positionen jeweils auch zu späteren Zeitpunkten insbesondere während einer Operation erneut betrachtet werden können.

Weiterhin ist es vorteilhaft, wenn der Abstand des Punkts zur Objektebene in der Steuereinheit als Parameter derart voreingestellt ist, dass er beispielsweise einen Wert im Bereich von 0 mm bis +/- 20 mm hat. Bei einem Abstand von +20 mm hat der Punkt einen um 20 mm größeren Abstand zum Endoskop als der Abstand der Objektebene zum Endoskop. Bei einem Abstand von -20 mm hat der Punkt einen um 20 mm kleineren Abstand zum Endoskop als der Abstand der Objektebene zum Endoskop. Hierdurch können ausgehend von der Position des Endoskops die auf die optische Achse des Endoskops und die Objektebene bezogenen Positionen einfach festgelegt werden. Vorzugsweise ist der Wert des voreingestellten Parameters von einem Benutzer änderbar, so dass dieser innerhalb des Bereichs von 0,001 mm bis 10 mm durch den Benutzer einstellbar und ggf. änderbar ist.

Ferner ist es vorteilhaft, wenn der Abstand des Punkts zur Objektebene derart in der Steuereinheit als Parameter voreingestellt ist, dass er innerhalb der Schärfentiefe liegt. Die Objektebene ist bei einem abbildenden optischen System einer Bilderfassungseinheit eine senkrecht zur optischen Achse verlaufende Ebene, die insbesondere den Objektpunkt enthält. Nur die Punkte der Objektebene werden scharf auf dem Bildsensor der Bilderfassungseinheit abgebildet. Die Schärfentiefe, die häufig auch als Tiefenschärfe bezeichnet wird, ist ein Maß für die Ausdehnung des scharfen Bereichs im Objektraum eines abbildenden optischen Systems. Die Schärfentiefe beschreibt die Größe des Entfernungsbereichs innerhalb dessen ein Objekt hinlänglich scharf abgebildet wird. Der Begriff Schärfentiefe ist beispielsweise in der DIN 19040 Blatt 3 definiert.

Somit ist der Bereich, in dem die zu ermittelnden Positionen liegen, innerhalb eines Bereichs beschränkt, in dem die Objekte noch hinreichend scharf darstellbar sind. Hierdurch erfolgt eine Beschränkung auf hinreichend scharf dargestellte Positionen, die für einen Betrachter gut erkennbar sind, so dass das Ermitteln von Positionen in nicht scharf sichtbaren Bereichen verhindert wird.

Ferner ist es vorteilhaft, wenn das Endoskop nach dem zweiten Bild zu einem dritten Zeitpunkt mindestens ein drittes Bild erfasst und ausgibt, wodurch die Position, Ausrichtung, Drehung, Lage und/oder Vergrößerung des Endoskops zwischen der Aufnahme des zweiten Bildes und des dritten Bildes unverändert sind. Hierbei gibt die Steuereinheit die auf dem ersten Abstandsvektor basierende Information zusammen mit dem dritten Bild aus. Da sich die Position des Endoskops zwischen der Aufnahme des zweiten und dritten Bildes nicht geändert hat, hat sich auch der erste Abstandsvektor nicht verändert, so dass dieser einfach zusammen mit dem dritten Bild ausgegeben werden kann. Ein erneutes Ermitteln des ersten Abstandsvektors ist hierbei nicht erforderlich.

Bei einer weiteren vorteilhaften Ausführungsform erfasst das Endoskop nach dem zweiten Bild mindestens ein weiteres viertes Bild zu einem vierten Zeitpunkt und gibt dieses aus. Die Position, Ausrichtung, Drehung, Lage und/oder Vergrößerung des Endoskops werden zwischen dem zweiten Zeitpunkt und dem vierten Zeitpunkt verändert. Die Steuereinheit ermittelt die Position eines Punkts der optischen Achse des Endoskops oder der zur optischen Achse des Endoskops parallelen Achse in der Objektebene. Alternativ ermittelt die Steuereinheit die Position eines Punkts der optischen Achse des Endoskops oder der zur optischen Achse des Endoskops parallelen Achse in einem Abstand zur Objektebene oder die Position der Strecke auf der optischen Achse des Endoskops oder der zur optischen Achse des Endoskops parallelen Achse. Die Position wird zu einem vierten Zeitpunkt als vierte Position ermittelt. Die Steuereinheit ermittelt einen zweiten Abstandsvektor zwischen der ersten Position und der vierten Position. Ferner gibt die Steuereinheit auf dem zweiten Abstandsvektor basierende Informationen zusammen mit einem zu oder nach dem vierten Zeitpunkt erfassten Bild aus. Dadurch wird bei einer Änderung der Lage des Endoskops bzw. einer Bildaufnahmeeigenschaft des Endoskops der Abstandsvektor neu ermittelt und der neu ermittelte Abstandsvektor als Grundlage für die zusammen mit dem weiteren aufgenommenen Bild auszugebenden Information ausgegeben. Somit kann in dem weiteren Bild ein aktueller Hinweis zum Wiederauffinden des Bezugspunkts ausgegeben werden.

Weiterhin ist es vorteilhaft, wenn die Steuereinheit prüft, ob die ermittelte erste Position in einem angezeigten Bild sichtbar ist. Das Endoskop hat hierbei das angezeigte Bild nach dem ersten Zeitpunkt erfasst. Die Steuereinheit führt eine optische Markierung an der ersten Position in das angezeigte Bild ein. Hierdurch ist ein leichtes Auffinden der als Bezugspunkt dienenden ersten Position möglich, da einem Betrachter durch die optische Markierung ein Hinweis zum Auffinden der ersten Position gegeben wird.

Weiterhin ist es vorteilhaft, wenn die Steuereinheit einen Vektorpfeil auf einem Teil einer Linie zwischen dem Mittelpunkt des angezeigten Bildes in Richtung der ersten Position erzeugt und in das angezeigte Bild einfügt. Hierdurch wird einem Betrachter ein klarer Hinweis gegeben, in welcher Richtung sich der Bezugspunkt befindet. Der Vektorpfeil selbst oder eine zusätzliche oder alternative Markierung kann auch in einem Randbereich des angezeigten Bildes eingefügt werden. Der Vektor selbst wird vorzugsweise in einem dreidimensionalen Koordinatensystem, wie einem dreidimensionalen Patientenkoordinatensystem oder einem dreidimensionalen Instrumentenkoordinatensystem ermittelt. Der Vektorpfeil wird vorzugsweise als dreidimensionaler Pfeil in eine dreidimensionale Bilddarstellung eingefügt. Hierdurch wird einem Betrachter ein leicht erfassbarer Hinweis gegeben, in welcher Richtung sich der Bezugspunkt ausgehend vom angezeigten Bild befindet, so dass dieser durch ein geeignetes Positionieren des Endoskops leicht wieder aufgefunden werden kann.

Dabei ist es vorteilhaft, wenn durch eine Benutzereingabe über eine Benutzerschnittstelle oder automatisch durch die Steuereinheit mindestens ein zweiter Bezugspunkt festgelegt wird. Die Steuereinheit kann dabei abhängig von einem voreingestellten Auswahlparameter wahlweise einen ersten Vektorpfeil und/oder einen zweiten Vektorpfeil erzeugen und in das angezeigte Bild einfügen. Der erste Vektorpfeil und/oder der zweite Vektorpfeil sind vorzugsweise in unterschiedliche Farben dargestellt oder haben unterschiedliche Markierungen, insbesondere unterschiedliche Farbmarkierungen. Hierdurch ist es einfach möglich, die Vektorpfeile dem jeweiligen Bezugspunkt zuzuordnen, so dass ein gewünschter Bezugspunkt einfach wieder auffindbar ist. Zusätzlich oder alternativ zu den Vektorpfeilen können auch andere Markierungen in das angezeigte Bild eingefügt werden.

Hierbei ist es ferner vorteilhaft, wenn die Steuereinheit ein Auswahlmenü mit anzeigbaren und/oder gespeicherten Bezugspunkten grafisch und/oder akustisch ausgibt. Über das Auswahlmenü sind anzeigbare Bezugspunkte auswählbar, d.h. Bezugspunkte, zu denen in das Bild Informationen eingefügt werden können. Ferner können über das Auswahlmenü auch zusätzliche oder alternative Hinweisfunktionen aktiviert und deaktiviert werden, beispielsweise eine akustische Information oder eine haptische Information. Alternativ oder zusätzlich sind anzeigbare und/oder gespeicherte Bezugspunkte mit Hilfe von Sprachbefehlen, die beispielsweise über ein Mikrofon eingebbar und von einer Steuereinheit verarbeitbar sind, auswählbar. Ferner können alternativ oder zusätzlich die anzeigbaren und/oder gespeicherten Bezugspunkte mit Hilfe von Körpergesten, die beispielsweise mit Hilfe einer Kamera erfasst und mit Hilfe einer Steuereinheit ausgewertet werden, insbesondere mit Hilfe von Fingergesten, die beispielsweise mit Hilfe einer tastsensitiven Oberfläche, wie einem Touchpad oder einem tastsensitiven Bildschirm, erfasst und mit Hilfe der Steuereinheit ausgewertet werden, ausgewählt werden.

Bei den erfindungsgemäßen Vorrichtungen oder einer der angeführten Weiterbildungen ist es ferner vorteilhaft, wenn die Steuereinheit vom Betrag des ermittelten Abstandsvektors abhängiges akustisches Signal oder eine vom Betrag des ermittelten Abstandsvektors abhängige visuelle Information oder eine haptische Information zur Signalisierung der Bewegungsrichtung zum Bezugspunkt hin über eine Handeingabevorrichtung einer Benutzerschnittstelle ausgibt. Die vom Betrag des ermittelten Abstandsvektors abhängige visuelle Information kann insbesondere über die Länge des angezeigten Vektorpfeils oder durch die Ausgabe des Betrags als Ziffernfolge, insbesondere mit einer Längeneinheit zur Spezifizierung des Abstands, ausgegeben werden. Als haptische Information zur Signalisierung der Bewegungsrichtung zum Bezugspunkt hin kann eine Gegenkraft, ein sogenanntes Force Feedback, implementiert werden. Als akustisches Signal kann eine Tonfolge mit einer abstandsabhängigen Frequenz der Abstände zwischen den Tönen der Tonfolge vorgesehen werden, insbesondere derart, dass bei einem großen Abstand zwischen den Punkten, d.h. bei einem großen Vektorbetrag die Töne der Tonfolge einen großen Abstand zueinander haben, wobei sich der Abstand bei einem geringen Betrag des Vektors verkleinert, vorzugsweise bis zur Ausgabe eines Dauertons.

Weiterhin ist es vorteilhaft, wenn die Steuereinheit einen ersten Bezugspunkt und ggf. einen weiteren Bezugspunkt jeweils als Bezugspunktmarkierung oder als Markierung eines am Bezugspunkt befindlichen Objekts in ein 3D-Modell eines zu operierenden Patienten einfügt. Dieses 3D-Modell kann dann zur Visualisierung und/oder Dokumentation des Operationsverlaufs genutzt werden. Das in der Nähe des Bezugspunkts befindliche Objekt kann beispielsweise ein Organ oder eine bestimmte Gewebestruktur sein, die dann vorzugsweise vollständig im 3D-Modell markiert wird.

Ferner ist es vorteilhaft, wenn das Endoskop ein Stereoendoskop mit einer gemeinsamen optischen Achse ist. Die Steuereinheit kann dann die erste, zweite, dritte und/oder vierte Position als Position eines Punkts der gemeinsamen optischen Achse des Stereoendoskops in der Objektebene oder als Position eines Punkts der optischen Achse des Stereoendoskops in einem Abstand zur Objektebene oder als Position einer Strecke auf der optischen Achse des Stereoendoskops mit einem Punkt in der Objektebene ermitteln. Alternativ kann das Endoskop ein Stereoendoskop mit zwei separaten Abbildungsoptiken, deren optischen Achsen parallel sind, sein, wobei die Steuereinheit die erste, zweite, dritte und/oder vierte Position als Position eines Punkts einer zwischen den optischen Achsen des Stereoendoskops verlaufenden Achse in der Objektebene oder als Position eines Punkts zwischen den optischen Achsen des Stereoendoskops verlaufende Achse in einem Abstand zur Objektebene oder Position einer Strecke auf der zwischen den optischen Achsen des Stereoendoskops verlaufende Achse mit einem Punkt in der Objektebene ermitteln. Die zwischen den optischen Achsen des Stereoendoskops verlaufende Achse verläuft vorzugsweise in der Mitte der optischen Achsen.

Somit kann die Erfindung auch einfach in Verbindung mit Stereoendoskopen eingesetzt werden.

Ein zweiter Aspekt der Erfindung betrifft ein System zur robotergestützten Chirurgie, insbesondere zu einer telerobotergestützten Prozedur mit einer erfindungsgemäßen Vorrichtung oder einer weiter oben angegebenen Weiterbildung, wobei die Benutzerschnittstelle mindestens eine Eingabeeinrichtung zur Eingabe mindestens eines Eingabekommandos umfasst. Die Steuereinheit und/oder eine weitere Steuereinheit steuert Aktoren des Systems zur robotergestützten Chirurgie derart an, dass das mit einem Manipulatorarm verbundene Endoskop und/oder ein mit einem weiteren Manipulatorarm verbundenes chirurgisches Instrument zur Gewebemanipulation abhängig von dem Eingabekommando mithilfe mindestens einer der Antriebseinheiten des Systems, vorzugsweise mithilfe mindestens einer Antriebseinheit des Manipulatorarms, positioniert wird. Hierdurch wird eine telerobotergestützte Prozedur bei einem chirurgischen Eingriff erleichtert. Insbesondere kann eine Bedienperson, beispielsweise ein Chirurg, einfach Bezugspunkte festlegen, die er dann mithilfe der erfindungsgemäßen Vorgehensweise einfach wiederauffinden kann. Hierdurch wird die Orientierung im Körper eines zu operierenden Patienten erheblich vereinfacht.

Ein dritter Aspekt betrifft ein Verfahren zum Festlegen und Wiederauffinden eines Bezugspunkts während eines chirurgischen Eingriffs, bei dem mithilfe eines Endoskops mehrere Bilder nacheinander als Bildfolge erfasst und den Bildern entsprechende Bilddaten erzeugt werden. Die Bilddaten werden mithilfe mindestens einer Steuereinheit verarbeitet und den Bilddaten entsprechende Bilder ausgegeben. Die Position eines Punkts der optischen Achse des Endoskops oder einer zur optischen Achse des Endoskops parallelen Achse in der Objektebene oder die Position eines Punkts der optischen Achse des Endoskops oder der zur optischen Achse des Endoskops parallelen Achse in einem Abstand zur Objektebene oder die Position einer Strecke auf der optischen Achse des Endoskops oder einer zur optischen Achse des Endoskops parallelen Achse mit einem Punkt in der Objektebene wird zu einem ersten Zeitpunkt als erste Position ermittelt. Die erste Position wird durch eine Bedieneingabe über eine Benutzerschnittstelle als erster Bezugspunkt festgelegt. Die Position eines Punkts der optischen Achse des Endoskops oder der zur optischen Achse des Endoskops parallelen Achse in der Objektebene oder die Position eines Punkts der optischen Achse des Endoskops oder der zur optischen Achse des Endoskops parallelen Achse in einem Abstand zur Objektebene oder die Position der Strecke auf der optischen Achse des Endoskops oder der zur optischen Achse des Endoskops parallelen Achse wird zu einem zweiten Zeitpunkt als zweite Position ermittelt. Zwischen der ersten Position und der zweiten Position wird ein erster Abstandsvektor ermittelt. Eine auf dem ersten Abstandsvektor basierende Information wird zusammen mit einem zu oder nach dem zweiten Zeitpunkt erfassten Bild ausgegeben.

Ein vierter Aspekt betrifft ein System zum Festlegen und Wiederauffinden eines Bezugspunkts während eines chirurgischen Eingriffs, bei dem mithilfe eines Endoskops mehrere Bilder nacheinander als Bildfolge erfasst und den Bildern entsprechende Bilddaten erzeugt werden. Mithilfe mindestens einer Steuereinheit werden die Bilddaten verarbeitet und den Bilddaten entsprechende Bilder ausgegeben. Die Position eines chirurgischen Instruments wird als erste Position ermittelt. Ferner wird die Position eines Punkts der optischen Achse des Endoskops oder einer zur optischen Achse des Endoskops parallelen Achse in der Objektebene oder die Position eines Punkts der optischen Achse des Endoskops oder der zur optischen Achse des Endoskops parallelen Achse in einem Abstand zur Objektebene oder Position einer Strecke auf der optischen Achse des Endoskops oder einer zur optischen Achse des Endoskops parallelen Achse mit einem Punkt in der Objektebene als zweite Position ermittelt. Zwischen der ersten Position und der zweiten Position wird ein erster Abstandsvektor ermittelt. Eine auf dem ersten Abstandsvektor basierende Information wird zusammen mit dem beim Ermitteln der ersten Position erfassten Bild ausgegeben.

Die Verfahren gemäß dem dritten und vierten Aspekt können in gleicher Weise weitergebildet werden wie für die Vorrichtungen oben angegeben.

Weitere Merkmale und Vorteile ergeben sich aus der folgenden Beschreibung, die die Erfindung anhand von Ausführungsbeispielen im Zusammenhang mit den beigefügten Figuren näher erläutert.

Es zeigen:
- Figur 1: eine schematische Seitenansicht eines Patienten und eines Systems zur robotergestützten Chirurgie mit einem Manipulator, der vier Manipulatorarme hat, mit denen jeweils eine Instrumenteneinheit verbindbar ist;
- Figur 2: eine schematische Vorderansicht des Patienten und des Systems nach Figur 1;
- Figur 3: einen Ausschnitt des Patienten und des Systems nach den Figuren 1 und 2;
- Figur 4: ein auf einer Anzeigeeinheit als Bild dargestelltes Sichtfeld zusammen mit einem außerhalb des Sichtfeldes befindlichen Instrument;
- Figur 5: ein auf einer Anzeigeeinheit als Bild dargestelltes Sichtfeld bei der Markierung eines Bezugspunktes; und
- Figur 6: ein auf einer Anzeigeeinheit als Bild dargestelltes Sichtfeld zusammen mit einem außerhalb des Sichtfeldes befindlichen Bezugspunkt.

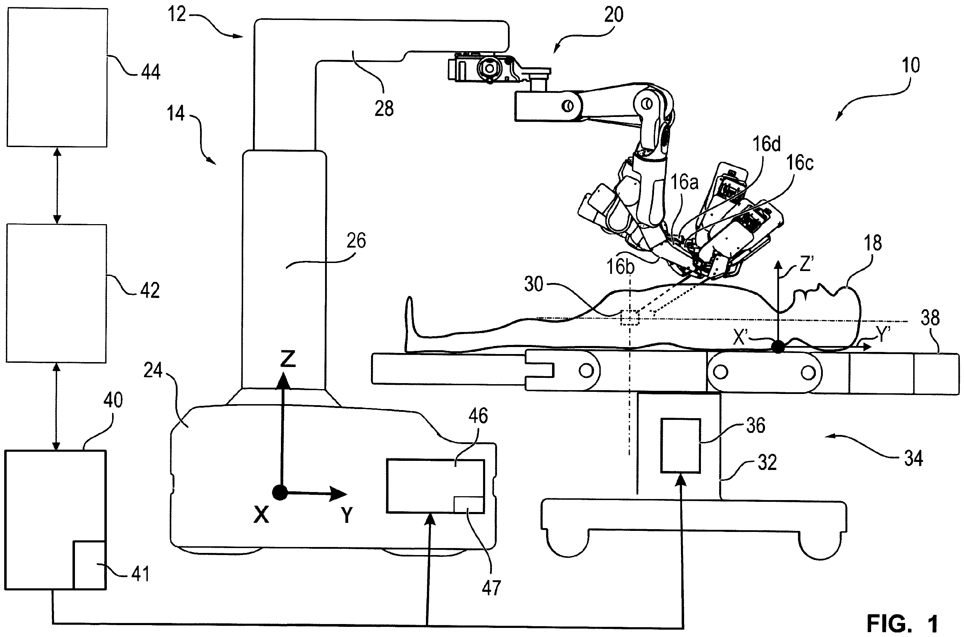

Figur 1 zeigt eine schematische Seitenansicht eines Patienten 18 und eines Systems 10 zur robotergestützten Chirurgie mit einem Manipulator 12, der ein Stativ 14 und vier Manipulatorarme 16a bis 16d hat. Der Manipulator 12 wird allgemein auch als Vorrichtung zur robotergestützten Chirurgie bezeichnet. Das System 10 dient zur Durchführung eines chirurgischen Eingriffs an einem auf einem Operationstisch 34 positionierten Patienten 18. Ausgehend von der Anatomie des Patienten 18 und der durchzuführenden Operation ergeben sich die Koordinaten x'_{z}, y'_{z}, z'_{z} eines Operationsgebiets 30 in einem Patientenkoordinatensystem X', Y', Z'. Der Manipulator 12 hat ein Koordinatensystem X, Y, Z der Vorrichtung 12, dessen Koordinatenursprung in einem Stativfuß 24 eines Stativs 14 des Manipulators 12 angeordnet ist. Das Stativ 14 hat eine fest mit dem Stativfuß 24 verbundene Traverse 26 mit der ein L-förmigen Stativarm 28 verbunden ist, an dessen vom Stativfuß 24 entfernten Ende die Manipulatorarme 16a bis 16d über einen Stativkopf 20 verbunden sind.

Der Operationstisch 34 hat eine Operationstischsäule 32, in der eine Steuereinheit 36 des Operationstischs 34 angeordnet ist und auf der eine mehrere Segmente umfassende Patientenlagerfläche 38 angeordnet ist. Die Steuereinheit 36 dient zur Steuerung der Bewegung von Elementen des Operationstischs 34, insbesondere zur Längenverstellung der Operationstischsäule 32 und somit zum Verstellen der Höhe der Patientenlagerfläche 38 und zum Verstellen einzelner Segmente sowie der Neigung und der Kantung der Patientenlagerfläche 38. Vorzugsweise ist jedoch die Verstellung der Segmente des Operationstischs 34 während eines operativen Eingriffs mit Hilfe des Manipulators 12 gesperrt. Das System 10 umfasst ferner eine Steuereinheit 46 des Manipulators 12 sowie eine zentrale Steuereinheit 40, die über Datenleitungen mit der Steuereinheit 46 des Manipulators 12, der Steuereinheit 36 des Operationstischs 34 sowie einer Bedienkonsole 42 mit einer Anzeigeeinheit 44 verbunden ist. Die Steuereinheit 40 hat eine Ausgabeeinheit 41 und die Steuereinheit 46 hat eine Ausgabeeinheit 47, durch die jeweils optische und/oder akustische Signale ausgegeben werden können.

Die Oberfläche der Patientenlagerfläche 38 bildet eine Frontalebene, auf der der Patient 18 positioniert ist. Ferner verläuft durch den Koordinatenursprung des Patientenkoordinatensystems X', Y', Z' eine Transversalebene, in der die Koordinatenachsen X' und Z' liegen. Ferner verläuft durch den Koordinatenursprung eine Medianebene, in der die Koordinatenachsen Z' und Y' liegen.

Die Koordinaten x'_{z}, y'_{z}, z'_{z} des Operationsgebiets 30 in dem Patientenkoordinatensystem X', Y', Z' sind bekannt und können durch die bekannte Lage des Patientenkoordinatensystems X', Y', Z' zum Koordinatensystem X, Y, Z der Vorrichtung 12 einfach bei der Ansteuerung der Manipulatorarme 16a bis 16d sowie der mit den Manipulatorarmen 16a bis 16d verbundenen Instrumenteneinheit zur Durchführung eines chirurgischen Eingriffs mit Hilfe des Manipulators 12 berücksichtigt werden, insbesondere in Koordinaten x_{z}, y_{z}, z_{z} des Koordinatensystems X, Y, Z der Vorrichtung 12 umgerechnet werden.

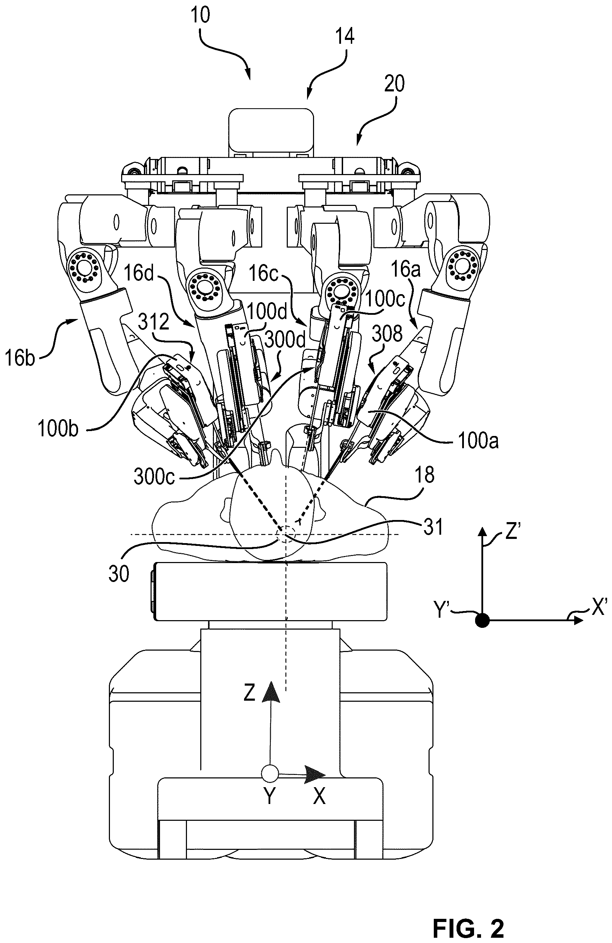

Figur 2 zeigt eine schematische Vorderansicht des Patienten 18 und des Systems 10 nach Figur 1. Am proximalen Ende der Manipulatorarme 16a bis 16d ist jeweils eine Koppeleinheit 100a bis 100d angeordnet, mit den jeweils eine Instrumenteneinheit 308 bis 300d zum Durchführen des chirurgischen Eingriffs verbunden ist. Die Instrumenteneinheiten 300c, 300d, 308, 312 sind mit Koppeleinheiten 100a, 100b, 100c, 100d der Manipulatorarme 16a bis 16d verbunden. Die Instrumentenschäfte der Instrumenteneinheiten 300c, 300d, 308, 312 sind zumindest mit ihrem vom Manipulatorarm 16a bis 16d entfernten Ende über Tokare in den Körper des Patienten 18 eingeführt und können mit Hilfe der Manipulatorarme 16a bis 16d so bewegt und positioniert werden, dass sie im Operationsgebiet 30 angeordnet sind. Dadurch können die an den von den Manipulatorarmen 16a, 16c und 16d entfernten Enden der Instrumentenschäfte vorhandenen Endeffektoren zur Gewebemanipulationen und andere zur Therapie erforderliche Handlungen im Operationsgebiet 30 genutzt werden. Auch die Spitze eines mit dem Manipulatorarm 16b verbundenen Endoskops 312 kann zur Erfassung von Bildern des Operationsgebiets 30 bis in das Operationsgebiet 30 bewegt werden. Mit Hilfe des Manipulatorarms 16b kann das Endoskop 312 so bewegt und positioniert werden, das Bilder unterschiedlicher Bereiche des Operationsgebietes 30 erfasst werden können, um einen Chirurgen eine gute Sicht auf die zur Behandlung relevanten Bereiche des Operationsgebietes 30 zu ermöglichen.

Mit Hilfe der durch das Operationsgebiet 30 verlaufenden und parallel zu den Koordinatenachsen X' und Z' verlaufenden Strichlinien sind die Koordinaten y'_{z}, z'_{z} des Mittelpunkts 31 des Operationsgebiets 30 in Bezug auf die Koordinatenachsen X' und Z' angedeutet. Auch wenn das Patientenkoordinatensystem X', Y' Z' in den Figuren 1 und 2 der Verständlichkeit halber zu dem Koordinatensystem X,Y,Z des Manipulators lediglich mit einer Linearverschiebung gezeichnet ist, können je nach Eingriff beide Koordinatensysteme auch beliebig zueinander ausgerichtet sein. Ihre Beziehung muss ferner nicht fest sein, sodass sich die Lage des Patientenkoordinatensystems X', Y' Z' während des Eingriffs ändern kann.

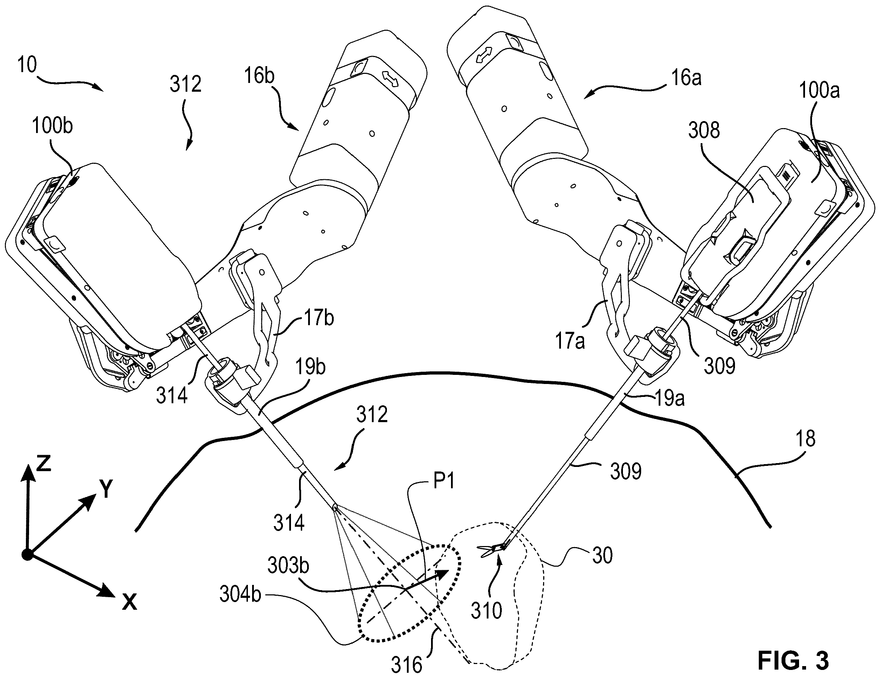

Figur 3 zeigt einen Teil des Patienten 18 und einen Ausschnitt des Systems 10 nach den Figuren 1 und 2. Konkret sind die instrumentenseitigen Armenden der Manipulatorarme 16a und 16b sowie eine schematische Schnittdarstellung des Patientenkörpers 18 mit dem relevanten Operationsbereich dargestellt. Mit der Koppeleinheit 100b des Manipulatorarms 16b ist eine Instrumenteneinheit 312 in Form eines Endoskops 312 verbunden. Das Endoskop 312 ist als Stabendoskop ausgeführt und dient zur Aufnahme von Bildern im Inneren des Körpers des Patienten 18. Das von der Koppeleinheit 100b entfernte Ende des Stabes 314 des Endoskops 312 ist durch einen mithilfe eines Trokarhalters 17b gehaltenen Trokars 19b in den Körper des Patienten 18 eingeführt und kann mithilfe des Manipulatorarms 16b in Folge von Bedieneingaben einer Bedienperson, insbesondere des Chirurgen, über eine Eingabeeinheit der Bedienkonsole 42 in seiner Lage relativ zum Patienten 18 geändert werden. Dadurch kann der Stab 314 insbesondere weiter in den Patientenkörper hinein sowie in entgegengesetzter Richtung bewegt werden. Auch der Winkel des Stabs 314 des Endoskops 312 kann durch eine entsprechende Bewegung des Manipulatorarms 16b verändert werden, insbesondere in Richtung des in Figur 3 dargestellten Pfeils P1, um so Bilder von weiteren Bereichen des Operationsgebiets 30 und insbesondere Bilder von den im Operationsgebiet 30 befindlichen Endeffektoren 310 aufzunehmen.

Der Endeffektor 310 der Instrumenteneinheit 308 ist an dem von der Koppeleinheit 100a entfernten Ende des Instrumentenschafts 309 der Instrumenteneinheit 308 angeordnet, wobei abhängig von den Bedieneingaben an der Bedienkonsole 42 sich die Instrumenteneinheit 308 so bewegen und steuern lässt, dass sowohl die Lage des Endeffektors 310 im Operationsgebiet 30 verändert werden kann, als auch verschiedene Bewegungen des Endeffektors 310 ausgeführt und Funktionen des Endeffektors 310 aktiviert werden können. Im vorliegenden Fall handelt es sich bei dem Endeffektor um eine Greifzange, die an dem Schaftende des Instrumentenschafts 309 um bis zu 90 Grad abwinkelbar ist. Es können jedoch anstatt der Instrumenteneinheit 308 auch alternative Instrumenteneinheiten mit der Koppeleinheit 100a verbunden werden. Der Endeffektor 310 kann aber auch ein monopolares chirurgisches Instrument zur Hochfrequenzchirurgie, das über die Koppeleinheit 100a und den Instrumentenschaft 309 mit elektrischer Energie versorgt wird, sein.

Der in die Körperöffnung des Patienten 18 durch den Trokar 19a einführbare Endeffektor 310 kann auch eine Klemme, eine Schere, ein Greifer, ein Nadelhalter, ein Mikrodissektor, eine Klammervorrichtung, eine Spül- und/oder Absaugvorrichtung, eine Schneidklinge, eine Kauterisationssonde, einen Katheter und/oder eine Saugdüse umfassen. Dadurch kann das durch die Instrumenteneinheit 308 bereitgestellte chirurgische Instrument wahlweise unterschiedliche Endeffektoren haben, die für gängige minimalinvasive Eingriffe, insbesondere bei der laparoskopischen Chirurgie, eingesetzt werden können. Es können jedoch auch andere chirurgische Instrumente zusätzlich oder alternativ eingesetzt werden. Insbesondere kann ein weiteres Endoskop und/oder eine zusätzliche Beleuchtungseinheit durch den Einsatz einer entsprechenden Instrumenteneinheit 308 vorgesehen werden, so dass diese dann bei dem chirurgischen Eingriff im Operationsgebiet 30 genutzt werden können.

Das Endoskop 312 ist über optische und/oder elektrische Übertragungsmittel mit der Koppeleinheit 100d des Manipulatorarms 16b verbunden. Diese können als Schnittstellen ausgeführt oder in Schnittstellen integriert sein. Die Übertragungsmittel dienen zur Kamerasteuerung und/oder Bilddatenübertragung und/oder Bildsignalübertragung. Auch können optische Lichtleiterfaserverbindungen vorgesehen sein, insbesondere um Beleuchtungslicht bis in den Operationsbereich 30 zu leiten. Der Schaft 309 des chirurgischen Instruments der Instrumenteneinheit 308 ist über einen durch einen Trokarhalter 17a am Manipulatorarm 16a gehaltenen Trokar 19a durch eine Köperöffnung des Patienten 18 in diesen eingeführt und bis in das Operationsgebiet 30 bewegt worden. Das Endoskop 312 hat im Inneren des Schafts 314 ein optisches Abbildungssystem, dessen optische Achse 316 durch die Strichpunktlinie in Figur 3 dargestellt ist. Dieses optische Abbildungssystem kann eine Festoptik oder eine Variooptik enthalten. Durch die Abbildungsoptik ist eine Objektebene definiert, die in Figur 3 durch eine im dreidimensionalen Raum platzierte Kreisdarstellung für den Bildaufnahmebereich des Endoskops 312 schematisch dargestellt und mit dem Bezugszeichen 304b bezeichnet ist. Die Objektebene 304b wird allgemein auch als Scharfstellebene bezeichnet. Der Schnittpunkt zwischen der optischen Achse und der Objektebene 304b ist in Figur 3 mit dem Bezugszeichen 303b bezeichnet. Wird das Endoskop 312 mithilfe des Manipulatorarms 16b so verschwenkt, dass die optische Achse 316 in Richtung des Pfeils P1 verschoben oder verschwenkt wird, so wird ein anderer Bereich des Operationsgebiets 30 mithilfe des Endoskops 312 erfasst, so dass das Endoskop 312 Bilder von diesem Bereich des Operationsgebiets 30 erfasst, die dann auf der Anzeigeeinheit 44 ausgegeben werden können. Wird das Endoskop 312 soweit verschwenkt, dass die optische Achse 316 die Pfeilspitze des Pfeils P1 tangiert, ist der Bilderfassungsbereich des Endoskops 312 so weit verschoben worden, dass sich auch der Endeffektor 310 des chirurgischen Instruments der Instrumenteneinheit 308 im Bilderfassungsbereich des Endoskops 312 befindet und ein Bild des Endeffektors 310 in einem durch das Endoskops 312 erfassten und auf der Anzeigeeinheit 44 ausgegebenen Bild sichtbar ist.

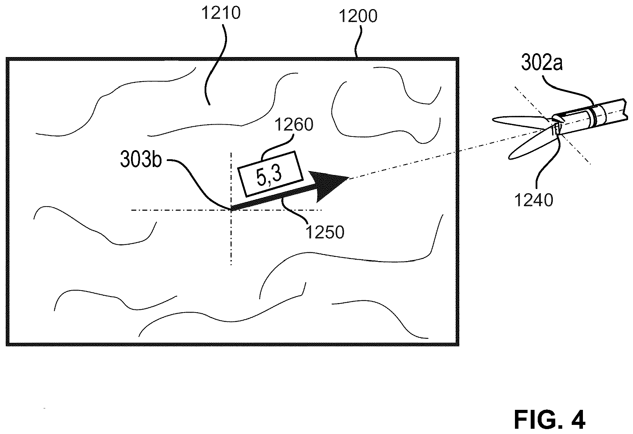

Figur 4 zeigt ein auf der Anzeigeeinheit 44 angezeigtes Bild 1200 des mithilfe des Endoskops 312 erfassten Sichtfeld. In dem angezeigten Bild 1200 sind die sichtbaren Gewebestrukturen 1210 im Operationsbereich 30 sowie weitere Informationen dargestellt. Mithilfe der sich kreuzenden Strichpunktlinien in der Mitte des angezeigten Bildes 1200 ist der Schnittpunkt 303b der optischen Achse 316 des Endoskops 312 mit der Objektebene 304b gezeigt. Der Endeffektor 310 befindet sich außerhalb des Sichtfeldes des Endoskops 312 und wird somit nicht in dem durch die Anzeigeeinheit 44 angezeigten Bild 1200 dargestellt. Ein Drehpunkt 1240 am vorderen Ende des abwinkelbaren Instrumentenschafts 309 wird als Bezugspunkt des Endeffektors 310 im vorigen Ausführungsbeispiel genutzt. Bei anderen Ausführungsbeispielen können auch anderer Punkte des Endeffektors als Bezugspunkt genutzt werden. Zwischen dem Schnittpunkt 303b und dem Bezugspunkt 1240 wird mithilfe der Steuereinheit 40 der Entfernungsvektor im dreidimensionalen Raum, vorzugsweise im dreidimensionalen Vorrichtungskoordinatensystem X, Y, Z des Manipulators 12 ermittelt. Der Betrag des Vektors wird optional als Entfernungsangabe 1260 in das angezeigte Bild 1200 eingefügt. Der dargestellte Pfeil 1250 verläuft in einem Abschnitt des Vektors. Der Vektor ist durch die Strecke zwischen dem Schnittpunkt 303b und dem Bezugspunkt 1240 festgelegt. Alternativ oder zusätzlich kann die Länge des dargestellten Pfeils 1250 von dem Betrag des Vektors abhängig sein. D.h., wenn der Endeffektor 310 eine größere Entfernung zum Sichtfeld des Endoskops 312 und somit eine größere Entfernung zum Schnittpunkt 303b hat, wird ein längerer Pfeil 1250 dargestellt und wenn die Entfernung geringer ist wird ein kürzerer Pfeil 1250 im angezeigten Bild 1200 dargestellt.

Somit ist mithilfe des dargestellten Pfeils 1250 das Auffinden des Endeffektors 310 im Operationsgebiet 30 einfach möglich, wenn der Endeffektor 310 selbst im mithilfe der Anzeigeeinheit 44 ausgegebenen Bild 1200 nicht sichtbar ist, da er sich in einem nicht angezeigten Bereich des mithilfe des Endoskops 312 erfassten Bildes oder außerhalb des Sichtbereichs des Endoskops 312 befindet.

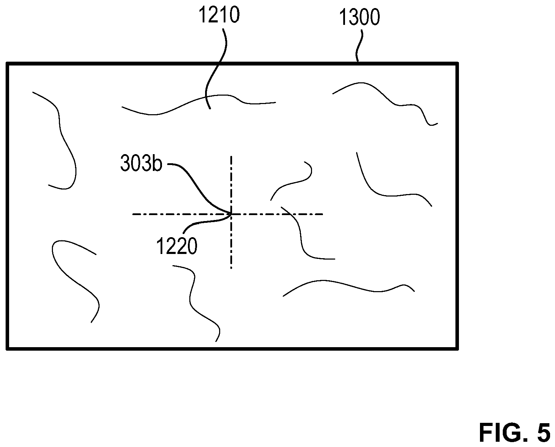

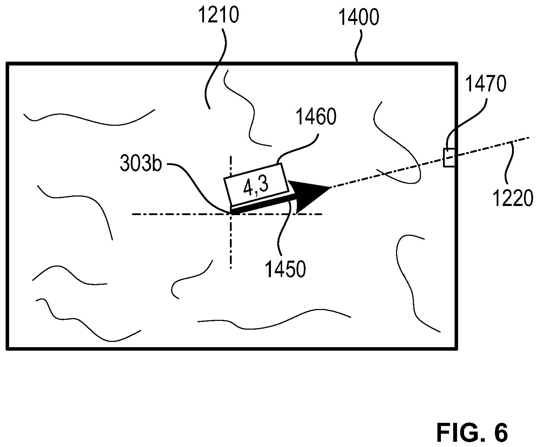

Figur 5 zeigt ein auf der Anzeigeeinheit 44 als Bild 1300 dargestelltes Sichtfeld des Endoskops 312. Die Position des Schnittpunkts 303b zwischen der optischen Achse des Endoskops 312 und der Objektebene 304b ist im Vorrichtungskoordinatensystem X, Y, Z über die kinematische Kette der Gelenke und Segmente des Manipulatorarms 16b eindeutig bestimmbar. Der Chirurg kann durch entsprechende Bedieneingaben über die Bedienkonsole 42 den Manipulatorarm 16b so bewegen, dass das Endoskop 312 so positioniert wird, dass der Schnittpunkt 303b an einer als Bezugspunkt 1220 zu markierenden Stelle des Operationsgebiets 30 liegt. Bei einer entsprechenden Bedieneingabe kann eine Bedienperson, insbesondere der Chirurg, diesen Punkt als Bezugspunkt markieren, wobei dessen Position gespeichert wird, so dass die Position zu einem späteren Zeitpunkt bei einer Aktivierung einer Funktion zum Wiederauffinden eines zuvor markierten Bezugspunkts 1220 zur Verfügung steht. Zusätzlich kann die Position dieses Bezugspunkts 1220 im Bild 1300 markiert werden und auch in weiteren dargestellten Bildern kann eine Markierung an der Stelle des gespeicherten Bezugspunkts 1220 eingefügt werde, so dass dieser für den Chirurgen im Bild 1300 gut erkennbar ist. Wird das Endoskop 312 anschließend so verschwenkt, dass der Bezugspunkt 1220 außerhalb des angezeigten Bildes 1400 angeordnet ist, wie dies in Figur 6 gezeigt ist, wird in dem in Figur 6 dargestellten angezeigten Bild 1400 ein Pfeil 1450 eingeblendet, der ausgehend von der Bildmitte die Richtung angibt, in der sich der Bezugspunkt 1220 ausgehend von der Bildmitte befindet, so dass dem Chirurgen ein Hinweis gegeben wird, wie er das Endoskop 312 verschwenken muss, damit der Bezugspunkt 1220 wieder in das Sichtfeld des Endoskops 312 gelangt bzw. wieder im angezeigten Bild 1400 bzw. in einem weiteren nachfolgend angezeigten Bild dargestellt wird. Durch solche Bezugspunkte 1220, von denen eine Vielzahl von Bezugspunkten 1220 durch den Chirurgen festlegbar ist, können die Operation wichtige Stellen im Situs des Patienten 18 einfach wiederaufgefunden werden, so dass die Orientierung des Chirurgen im Operationsbereich 30 erleichtert ist, was insbesondere zu einem verbesserten Operationsablauf führt, wodurch die Sicherheit des Patienten 18 erhöht und die Operationszeit verkürzt werden kann.

Ist das Endoskop 312 ein Stereoendoskop, können die Bilder 1200, 1300 und 1400 auch als dreidimensionale Bilder erfasst und ausgegeben werden Die Pfeile 1250 und 1450 können dann mit einer entsprechenden Ausrichtung entlang des Verlaufs des ermittelten Vektors in das dreidimensionale Bild 1200, 1300, 1400 so eingefügt werden, dass auch die Darstellung der Pfeile 1250 und 1450 dreidimensional erfolgt und damit der Chirurg eine echte dreidimensionale Richtungsinformation erhält. Auch bei den Bezugspunkten 1220 nach den Figuren 5 und 6 kann zusätzlich zum Richtungspfeil 1450 eine Zahlenwertangabe 1460 zum Spezifizieren der Entfernung des Schnittpunkts 303b zum Bezugspunkt 1220 dargestellt werden. Besonders vorteilhaft ist es, wenn über einer Voreinstellung festgelegt werden kann, ob ein Zahlenwert 1260, 1460 zusätzlich zum Richtungspfeil 1250, 1450 angezeigt werden soll oder nicht. Alternativ zu den Darstellungen in den Figuren 4 und 6 kann der Richtungspfeil 1250, 1450 auch in einem Randbereich des angezeigten Bildes 1200 bzw. 1400 angezeigt werden, so dass der Chirurg in der Mitte des Bildes 1200, 1300 einen freien Sichtbereich hat. Somit wird sowohl bei der Ausführungsform nach Figur 4 als auch nach der Ausführungsform nach den Figuren 5 und 6 jeweils ein Abstandsvektor eines durch das Endoskop 312 spezifizierten Punkts 303b und einem weiteren Punkt im Operationsbereich 30, wie den Bezugspunkt 1240 des Endeffektors 310 oder einem zuvor festgelegten Bezugspunkt 1220. Aufgrund der bekannten kinematischen Struktur der Manipulatorarme 16a bis 16d und den mit den Manipulatorarmen verbundenen Instrumenteneinheiten 308, 300c, 300d sowie dem mit dem Manipulatorarm 16b verbundenen Endoskop 312 kann die Position des Punktes 303b sowie des Punktes 1240 sowie Bezugspunkte anderer Instrumenteneinheiten 300c, 300d jederzeit einfach ermittelt werden. Eine visuelle Darstellung eines den Abstandsvektor visualisierenden Pfeils 1250, 1450 als zweidimensionale Darstellung in einem zweidimensional angezeigten Bild 1200, 1300, 1400 oder als dreidimensionaler Pfeil in einem dreidimensionalen Bild 1200, 1300, 1400 dient als Orientierungshilfe beim Bewegen des Endoskops 312, um den Instrumentenbezugspunkt 1240 bzw. den Bezugspunkt 1220 in den Sichtbereich des Endoskops 312 zu bringen und damit im angezeigten Bild 1200, 1300, 1400 anzeigen zu können. Zusätzlich oder alternativ zu den in das Bild eingefügten Pfeil 1250, 1450 kann eine akustische Signalisierung über die Ausgabeeinheiten 47, 41 erfolgen, um beim Bewegen des Endoskops 312 zu signalisieren, ob sich der Sichtbereich des Endoskops 312 dem Bezugspunkt 1220, 1240 annähert oder von diesem entfernt. Ferner ist zusätzlich oder alternativ eine haptische Unterstützung zur Signalisierung der richtigen Bewegungsrichtung bei einer Handeingabe über eine sogenannte Force Feedbackfunktion möglich, um bei einer Bewegung des Endoskops 312 die Handeingabe so zu führen, dass der Bezugspunkt 1220, 1240 in den Sichtbereich des Endoskops 312 kommt. Das Festlegen von Bezugspunkten 1220 im Operationsbereich 30 dient insbesondere zum Festlegen von sogenannten anatomischen Landmarken. Hierbei kann es sich insbesondere um für die Operation oder allgemein zur Orientierung relevanter Gewebestrukturen oder Organe im Operationsbereich 30 handeln, die mithilfe der beschriebenen Methode als Bezugspunkt 1220 markiert werden. Hierbei ist es insbesondere möglich mithilfe von entsprechenden Mustererkennungsverfahren zusammenhängende Gewebestrukturen, insbesondere Organe, zu erkennen und diese Gewebestrukturen bzw. Organe insgesamt im angezeigten Bild 1200, 1300 1400 mithilfe einer geeigneten Markierung zu kennzeichnen. Hierzu können die Gewebestrukturen bzw. die Organe im angezeigten Bild 1200, 1300, 1400 beispielsweise eingefärbt oder umrandet werden.

Durch die in Verbindung mit den Figuren 1 bis 6 beschriebene Vorgehensweise kann die Position von Bezugspunkten 1220, 1240 und die Position sowie die Orientierung von chirurgischen Instrumenten und Endeffektoren 310 in Bezug auf das Sichtfeld des Endoskops 312 bzw. in Bezug auf das angezeigte Bild 1200, 1300, 1400 einer Visualisierungsvorrichtung, wie der Anzeigeeinheit 44, einfach ermittelt werden, da über die kinematische Kette der Manipulatorarme 16a bis 16d sowohl die exakte Lage und Ausrichtung des Endoskops 312 als auch die exakte Lage und Ausrichtung der Endeffektoren 310 der Instrumenteneinheiten 308 bekannt sind. Die Positionen und Orientierung werden jeweils in Bezug auf das Vorrichtungskoordinatensystem XYZ des Manipulators 12 erfasst. Die Manipulatorarme 16a bis 16d umfassen eine Vielzahl von Gelenken, welche durch starre Segmente miteinander verbunden sind. Die Schwenkachsen der Gelenke sind dabei so verschieden im Raum positioniert, dass mithilfe von mindestens drei Gelenken in einem für die Operation sinnvollen Arbeitsbereich eine beliebige erforderliche Position und Ausrichtung sowohl des Endoskops 312 als auch der Endeffektoren 310 möglich ist.

Die Manipulatorarme 16a bis 16d sind, wie bereits in Verbindung mit Figur 1 beschrieben, über den Stativkopf 20 mit dem Stativ 14 verbunden, so dass der Stativkopf 20 während der Operation in gleicher Weise wie das Stativ 14 selbst eine ortsfeste Basis bietet. Ausgehend von dieser gemeinsamen Basis kann für jedes Gelenk durch eine entsprechende Gelenksensorik der aktuelle Konfigurationszustand des Gelenks ermittelt und in der zentralen Steuereinheit 40 erfasst und verarbeitet werden. Die Gelenksensorik, kann insbesondere jeweils einen Drehwinkelencoder umfassen. Alternativ zur Gelenksensorik können auch Schrittmotoren eingesetzt werden, deren Position über die Schrittansteuerung des Schrittmotors exakt bestimmbar ist. Somit ist über die Gelenksensorik bzw. die bekannte Ansteuerung der Antriebseinheiten zum Verschwenken der Segmente um die Gelenke herum die kinematische Kette der Manipulatorarme 16a bis 16d exakt bestimmbar. Insbesondere ist die Länge der Segmente, die Orientierung der Gelenke in Bezug auf die angrenzenden Segmente sowie die Winkelstellung und Orientierung der Gelenke in Bezug auf das Sichtfeld des Endoskops 312 bekannt.

Auch die Lage und Orientierung des Sichtfelds bzw. der Objektebene 304b des Endoskops 312 kann über die kinematische Kette des Manipulatorarms 16b in gleicher Weise bestimmt werden, wie Position und Ausrichtung der Endeffektoren 310 der Manipulatorarme 16a, 16c und 16d. Somit kann sowohl die Lage des Schnittpunkts 303b als auch die Lage der Bezugspunkte 1240 der Endeffektoren 310 oder anderer Instrumente oder Hilfsmittel jederzeit einfach und sicher berechnet und für eine entsprechende Informationsausgabe, wie über die Pfeile 1250, 1450, genutzt werden. Somit sind die Abstandsvektoren im dreidimensionalen Raum zwischen dem Schnittpunkt 303b und einem beliebigen Bezugspunkt 1220, 1240 im dreidimensionalen Raum, insbesondere im Operationsbereich 30, möglich.

Im Falle einer visuellen Informationsausgabe kann die Darstellung der Information beispielsweise durch das Anheften eines Vektorpfeils an den im Bild 1200, 1400 dargestellten Schnittpunkt 303b erfolgen und insbesondere durch die räumliche Darstellung eines Vektorpfeils in Richtung des Bezugspunkts 1200, 1240. Bei der Nutzung eines dreidimensionalen Displays kann dieser Vektorpfeil 1250 in Richtung des Bezugspunkts 1240, 1220 zeigen, der zuvor ausgewählt worden ist.

Bei alternativen Ausführungsbeispielen können auch mehrere Bezugspunkte festgelegt sein, so dass dann mehrere Vektorpfeile 1250, 1450 in das angezeigte Bild 1200, 1300, 1400 eingefügt werden. Die angezeigten Pfeile 1250, 1450 haben dann vorzugsweise unterschiedliche Farben, wobei jeweils eine Farbe einem zuvor festgelegten Bezugspunkt zugeordnet ist. Hierdurch wird die Orientierung weiter erleichtert, so dass dem Chirurgen eine Information gegeben wird, welcher Pfeil eine Information zu welchem Bezugspunkt gibt.

Die Länge des dargestellten Vektorpfeils 1250 kann zusätzlich oder alternativ zur Angabe des Zahlenwertes dazu genutzt werden, den Betrag des Abstands des Schnittpunkts 303b vom Bezugspunkt 1220, 1240 anzugeben, wie dies durch die unterschiedlich langen Pfeile 1250, 1450 in den Figuren 4 und 6 erfolgt ist. Im Fall einer akustischen Informationsausgabe kann der Betrag des Abstandsvektors zwischen dem Schnittpunkt 303b und dem Bezugspunkt 1220, 1240 durch den Abstand einzelner Töne oder durch die Frequenz eines Dauertons oder durch die Kombination des Abstands der Töne einer Tonfolge und der Frequenz der Töne einer Tonfolge signalisiert werden. Bei der Nutzung von Stereo- oder 3D-Lautsprechern bzw. Stereo- oder 3D-Kopfhörern kann durch eine Verschiebung des Pegels zwischen den Kanälen eine weitere räumliche Information ausgegeben werden. Dies kann insbesondere in ähnlicher Weise erfolgen, wie bei bekannten Einparkhilfen bei Personenkraftwagen.

Im Falle einer haptischen Informationsausgabe kann die Rückmeldung an den Chirurgen durch die Erzeugung von Kräften auf Eingabeelemente eines haptischen Eingabegeräts derart erfolgen, dass eine Bewegung des Endoskops 312 in einer Richtung, in der sich der Sichtbereich dem Bezugspunkt 1220, 1240 derart nähert, dass der Bezugspunkt 1220, 1240 zumindest bei einer weiteren Verschiebung des Sichtbereichs, in den Sichtbereich gelangt, keine oder nur eine geringe Gegenkraft auf die Eingabeelemente wirkt und bei einer Entfernung des Sichtbereichs vom Bezugspunkt 1220, 1240 größere Kräfte auf die Eingabeelemente wirken, so dass die Führung der Hand des Chirurgen von dem Eingabegerät der Bedienkonsole 42 erfolgt, dass er das Endoskop 312 intuitiv so durch den Manipulatorarm 16b bewegt, dass der Abstandsvektor zwischen dem Schnittpunkt 303b und dem Bezugspunkt 1220, 1240 kleiner wird.

### Bezugszeichenliste

- 10: System
- 12: Manipulator
- 14: Stativ
- 16a bis 16d: Manipulatorarm
- 17a, 17b: Trokar-Halterung
- 18: Patient
- 19a, 19b: Trokar
- 20: Stativkopf
- 24: Stativfuß
- 26: Traverse
- 28: Stativarm
- 30: Zieloperationsgebiet
- 31: Mittelpunkt des Zieloperationsgebiet
- 32: Operationstischsäule
- 34: Operationstisch
- 36: Steuereinheit des Operationstisches
- 38: Patientenlagerfläche
- 40: zentrale Steuereinheit der Vorrichtung
- 41: Ausgabeeinheit
- 42: Bedienkonsole
- 44: Anzeigeeinheit
- 46: Steuereinheit des Manipulators
- 47: Ausgabeeinheit
- 100, 100a bis 100d: Koppeleinheit
- 300, 300c, 300d, 308, 312: Instrumenteneinheit
- 309: Instrumentenschaft
- 314: Endoskopschaft
- 308: Endeffektor
- 316: optische Achse
- 303b: Schnittpunkt
- 304b: Objektebene
- 312: Endoskop
- 1200, 1300, 1400: angezeigtes Bild
- 1220,1240: Bezugspunkt
- 1250,1450: Vektorpfeil
- 1260,1460: Zahlenwert
- P1: Richtungspfeil
- X, Y, Z: Koordinatensystem der Vorrichtung
- X', Y', Z': Patientenkoordinatensystem

## Patentansprüche

1. Vorrichtung zum Festlegen und Wiederauffinden eines Bezugspunktes während eines chirurgischen Eingriffs,
mit einem Endoskop (312), das mehrere Bilder nacheinander als Bildfolge erfasst und den Bildern entsprechende Bilddaten erzeugt, mit mindestens einer Steuereinheit (40) und mindestens einer Anzeigeeinheit (44),
wobei die mindestens eine Steuereinheit (40) die Bilddaten verarbeitet und den Bilddaten entsprechende Bilder (1200, 1300, 1400) auf der mindestens einen Anzeigeeinheit (44) ausgibt,
wobei die Steuereinheit (40) dazu ausgelegt ist, die Position eines Punkts der optischen Achse des Endoskops (312) oder einer zur optische Achse (316) des Endoskops (312) parallelen Achse in der Objektebene (304b) oder die Position einer Strecke auf der optischen Achse (316) des Endoskops (312) oder einer zur optische Achse (316) des Endoskops (312) parallelen Achse mit einem Punkt (303b) in der Objektebene (304b) zu einem ersten Zeitpunkt als erste Position zu ermitteln,
wobei die erste Position durch eine Bedieneingabe über eine Benutzerschnittstelle als erster Bezugspunkt (1220) festlegbar ist,
wobei die Steuereinheit (40) weiterhin dazu ausgelegt ist, die Position eines Punktes der optischen Achse (316) des Endoskops (312) oder der zur optische Achse (316) des Endoskops (312) parallelen Achse in der Objektebene (304b) oder die Position der Strecke auf der optischen Achse (316) des Endoskops (312) oder der zur optische Achse (316) des Endoskops (312) parallelen Achse zu einem zweiten Zeitpunkt als zweite Position (303b) zu ermitteln,
wobei die Steuereinheit (40) weiterhin dazu ausgelegt ist, einen ersten Abstandsvektor zwischen der ersten Position (1220) und der zweiten Position (303b) zu ermitteln,
wobei die Steuereinheit (40) eine auf dem ersten Abstandsvektor basierende Information (1450, 1560) zusammen mit einem zu oder nach dem zweiten Zeitpunkt erfassten Bild (1400) auf der Anzeigeeinheit (44) ausgibt.

2. Vorrichtung nach Anspruch 1, **dadurch gekennzeichnet, dass** die Steuereinheit (40) einen Bildausschnitt der Umgebung der ermittelten ersten Position beim Erfassen der ersten Position (1220) erfasst und zum Wiederauffinden der ersten Position (1220) in einem nach dem Ermitteln der ersten Position (1220) erfassten weiteren Bild (1400) mit mindestens einem Bildausschnitt des weiteren Bildes vergleicht.

3. Vorrichtung nach einem der vorhergehenden Ansprüche, **dadurch gekennzeichnet, dass** die Steuereinheit (40) die erste Position (1220, 1240) ausgehend von einem mit Hilfe des Endoskops (312) erfassten ersten Bildes (1200, 1300, 1400) oder ausgehend von der Position des Endoskops (312) zum ersten Zeitpunkt ermittelt, und
dass die Steuereinheit (40) die zweite Position (303b) ausgehend von einem nach dem ersten Bild (1200, 1300, 1400) mit Hilfe des Endoskops (312) erfassten zweiten Bildes (1200, 1300, 1400) oder ausgehend von der Position des Endoskops (312) zum zweiten Zeitpunkt ermittelt.

4. Vorrichtung nach einem der vorhergehenden Ansprüche, **dadurch gekennzeichnet, dass** die Steuereinheit (40) die Positionen jeweils mit Hilfe der kinematischen Kette eines mit dem Endoskop (312) verbundenen Manipulatorarms (16b) eines chirurgischen Robotersystems (10) ermittelt.

5. Vorrichtung nach einem der vorhergehenden Ansprüche, **dadurch gekennzeichnet, dass** das Endoskop (312) zum ersten Zeitpunkt ein erstes Bild (1200, 1300, 1400) erfasst, und dass das Endoskop (312) zum zweiten Zeitpunkt ein zweites Bild (1200, 1300, 1400) erfasst, oder
dass das Endoskop (312) vor oder beim Ermitteln der ersten Position ein erstes Bild (1200, 1300, 1400) erfasst und dass das Endoskop (312) vor oder beim Ermitteln der zweiten Position ein zweites Bild (1200, 1300, 1400) erfasst.

6. Vorrichtung nach einem der vorhergehenden Ansprüche, **dadurch gekennzeichnet, dass** das Endoskop (312) nach dem zweiten Bild (1200, 1300, 1400) zu einem dritten Zeitpunkt mindestens ein weiteres drittes Bild (1200, 1300, 1400) erfasst und ausgibt, wobei die Position, Ausrichtung, Drehung, Lage und/oder Vergrößerung des Endoskops (312) zwischen der Aufnahme des zweiten Bildes (1200, 1300, 1400) und des dritten Bildes (1200, 1300, 1400) unverändert sind,
wobei die Steuereinheit (40) die auf dem ersten Abstandsvektor basierende Information zusammen mit dem dritten Bild (1200, 1300, 1400) ausgibt.

7. Vorrichtung nach einem der vorhergehenden Ansprüche, **dadurch gekennzeichnet, dass** das Endoskop (312) nach dem zweiten Bild (1200, 1300, 1400) mindestens ein weiteres viertes Bild (1200, 1300, 1400) zu einem vierten Zeitpunkt erfasst und ausgibt,
wobei die Position, Ausrichtung, Drehung, Lage und/oder Vergrößerung des Endoskops (312) zwischen dem zweiten Zeitpunkt und dem vierten Zeitpunkt verändert sind,
wobei die Steuereinheit (40) die Position eines Punktes (303b) der optischen Achse (316) des Endoskops (312) oder der zur optische Achse (316) des Endoskops (312) parallelen Achse in der Objektebene (304b) oder die Position eines Punkts der optischen Achse (316) des Endoskops (312) oder der zur optische Achse (316) des Endoskops (312) parallelen Achse in dem Abstand zur Objektebene (304b) oder die Position der Strecke auf der optischen Achse (316) des Endoskops (312) oder der zur optische Achse (316) des Endoskops (312) parallelen Achse zu einem vierten Zeitpunkt als vierte Position (303b) ermittelt,
wobei die Steuereinheit (40) einen zweiter Abstandsvektor zwischen der ersten Position (1220, 1240) und der vierten Position (303b) ermittelt,
wobei die Steuereinheit (40) eine auf dem zweiten Abstandsvektor basierende Information (1250, 1260, 1450, 1460) zusammen mit einem zu oder nach dem vierten Zeitpunkt erfassten Bild (1200, 1300, 1400) ausgibt.

8. Vorrichtung nach einem der vorhergehenden Ansprüche, **dadurch gekennzeichnet, dass** die Steuereinheit (40) prüft, ob die ermittelte erste Position (1220) in einem angezeigten Bild (1200, 1300, 1400) sichtbar ist,
wobei das Endoskop (312) das angezeigte Bild (1200, 1300, 1400) nach dem ersten Zeitpunkt erfasst hat, und
wobei die Steuereinheit (40) eine optische Markierung an der ersten Position (1220) in das angezeigte Bild (1200, 1300, 1400) einfügt.

9. Vorrichtung nach einem der vorhergehenden Ansprüche, **dadurch gekennzeichnet, dass** die Steuereinheit (40) einen Vektorpfeil (1250, 1450) auf einem Teil einer Linie zwischen dem Mittelpunkt des angezeigten Bildes (1200, 1300, 1400) in Richtung der ersten Position erzeugt und in das angezeigte Bild (1200, 1300, 1400) einfügt.

10. Vorrichtung nach Anspruch 9, **dadurch gekennzeichnet, dass** durch eine Benutzereingabe über eine Benutzerschnittstelle (42) oder automatisch durch die Steuereinheit (40) mindestens ein zweiter Bezugspunkt festgelegt wird, dass die Steuereinheit (40) abhängig von einem voreingestellten Auswahlparameter wahlweise einen ersten Vektorpfeil (1250) und/oder einen zweiten Vektorpfeil (1450) erzeugt und in das angezeigte Bild (1200, 1300, 1400) einfügt.

11. Vorrichtung nach einem der vorhergehenden Ansprüche, **dadurch gekennzeichnet, dass** die Steuereinheit (40) ein vom Betrag des ermittelten Abstandsvektors abhängiges akustisches Signal oder eine vom Betrag des ermittelten Abstandsvektors abhängige visuelle Information oder eine haptische Information zur Signalisierung der richtigen Bewegungsrichtung über eine Handeingabevorrichtung einer Benutzerschnittstelle (42) ausgibt.

12. Vorrichtung nach einem der vorhergehenden, **dadurch gekennzeichnet, dass** die Steuereinheit (40) den ersten Bezugspunkt (1220) und ggf. einen weiteren Bezugspunkt jeweils als Bezugspunktmarkierung oder als Markierung eines am Bezugspunkt (1220) befindlichen Objekts in ein 3D-Modell eines zu operierenden Patienten (18) einfügt.

13. Vorrichtung nach einem der vorhergehenden Ansprüche, **dadurch gekennzeichnet, dass** das Endoskop (312) ein Stereoendoskop mit einer gemeinsamen optischen Achse (316) ist, wobei die Steuereinheit (40) die jeweilige Position als Position eines Punkts der gemeinsamen optischen Achse (316) des Stereoendoskops in der Objektebene (304b) oder als Position eines Punkts der optischen Achse (316) des Stereoendoskops in einem Abstand zur Objektebene (304b) oder als Position einer Strecke auf der optischen Achse (316) des Stereoendoskops mit einem Punkt (303b) in der Objektebene (304b) ermittelt, oder
dass das Endoskop (312) ein Stereoendoskop mit zwei separaten Abbildungsoptiken, deren optische Achsen parallel sind, ist, wobei die Steuereinheit (40) die jeweilige Position als Position eines Punkts einer zwischen den optischen Achsen des Stereoendoskops verlaufenden Achse in der Objektebene (304b)oder als Position eines Punkts einer zwischen den optischen Achsen des Stereoendoskops verlaufenden Achse in einem Abstand zur Objektebene (304b) oder als Position einer Strecke auf der zwischen den optischen Achsen des Stereoendoskops verlaufende Achse mit einem Punkt (303b) in der Objektebene (304b) ermittelt.

14. System zur robotergestützten Chirurgie, insbesondere zu einer telerobotergestützten Prozedur, mit einer Vorrichtung (12) nach einem der vorhergehenden Ansprüche, wobei das System weiterhin die Benutzerschnittstelle (42) umfasst, wobei die Benutzerschnittstelle (42) mindestens eine Eingabeeinrichtung zur Eingabe mindestens eines Eingabekommandos umfasst,
wobei die mindestens eine Steuereinheit (40) oder eine weitere Steuereinheit (40) Aktoren des Systems (10) zur robotergestützten Chirurgie derart ansteuert, dass das mit einem Manipulatorarm (16b) verbundene Endoskop (312) und/oder ein mit einem weiteren Manipulatorarm (16a) verbundenes chirurgisches Instrument (308, 310) zur Gewebemanipulation abhängig von dem Eingabekommando mit Hilfe mindestens einer Antriebseinheit positioniert wird.

## Claims

1. A device for setting and retrieving a reference point during a surgical operation,
with an endoscope (312) which captures several images successively as an image sequence and generates image data corresponding to the images, with at least one control unit (40) and at least one display unit (44), wherein the at least one control unit (40) processes the image data and outputs images (1200, 1300, 1400) corresponding to the image data on the at least one display unit (44),
wherein the control unit (40) is configured to determine the position of a point of the optical axis of the endoscope (312) or of an axis parallel to the optical axis (316) of the endoscope (312) in the object plane (304b), or the position of a line segment on the optical axis (316) of the endoscope (312) or on an axis parallel to the optical axis (316) of the endoscope (312) with a point (303b) in the object plane (304b) at a first point in time as a first position, the first position being settable by a user input via a user interface as a first reference point (1220),
wherein the control unit (40) is further configured to determine the position of a point of the optical axis (316) of the endoscope (312) or of the axis parallel to the optical axis (316) of the endoscope (312) in the object plane (304b), or the position of the line segment on the optical axis (316) of the endoscope (312) or on the axis parallel to the optical axis (316) of the endoscope (312) at a second point in time as a second position (303b),
wherein the control unit (40) is further configured to determine a first distance vector between the first position (1220) and the second position (303b),
wherein the control unit (40) outputs on the display unit (44) an information (1450, 1560) based on the first distance vector together with an image (1400) captured at or after the second point in time.

2. The device according to claim 1, **characterized in that** the control unit (40) captures an image detail of the surrounding of the determined first position when capturing the first position (1220) and, for retrieving the first position (1220), compares it in a further image (1400) captured after determining the first position (1220) with at least an image detail of the further image.

3. The device according to one of the preceding claims, **characterized in that** the control unit (40) determines the first position (1220, 1240) based on a first image (1200, 1300, 1400) captured by the endoscope (312) or based on the position of the endoscope (312) at the first point in time, and
that the control unit (40) determines the second position (303b) based on a second image (1200, 1300, 1400) captured by the endoscope (312) after the first image (1200, 1300, 1400) or based on the position of the endoscope (312) at the second point in time.

4. The device according to one of the preceding claims, **characterized in that** the control unit (40) determines the positions each time via the kinematic chain of a manipulator arm (16b) of a surgical robot system (10) connected to the endoscope (312).

5. The device according to one of the preceding claims, **characterized in that** the endoscope (312) captures a first image (1200, 1300, 1400) at a first point in time, and that the endoscope (312) captures a second image (1200, 1300, 1400) at the second point in time, or
that the endoscope (312) captures a first image (1200, 1300, 1400) before or upon determination of the first position and that the endoscope (312) captures a second image (1200, 1300, 1400) before or upon determination of the second position.

6. The device according to one of the preceding claims, **characterized in that** after the second image (1200, 1300, 1400) the endoscope (312) captures and outputs at a third point in time at least a further third image (1200, 1300, 1400), wherein the position, orientation, rotation, location and/or magnification of the endoscope (312) between capturing the second image (1200, 1300, 1400) and the third image (1200, 1300, 1400) are unchanged,
wherein the control unit (40) outputs the information based on the first distance vector together with the third image (1200, 1300, 1400).

7. The device according to one of the preceding claims, **characterized in that** after the second image (1200, 1300, 1400) the endoscope (312) captures and outputs at least a further fourth image (1200, 1300, 1400) at a fourth point in time, and
wherein the position, orientation, rotation, location and/or magnification of the endoscope (312) between the second point in time and the fourth point in time are changed,
wherein the control unit (40) determines the position of a point (303b) of the optical axis (316) of the endoscope (312) or of the axis parallel to the optical axis (316) of the endoscope (312) in the object plane (304b), or the position of a point of the optical axis (316) of the endoscope (312) or of the axis parallel to the optical axis (316) of the endoscope (312) at the distance to the object plane (304b), or the position of the line segment on the optical axis (316) of the endoscope (312) or on the axis parallel to the optical axis (316) of the endoscope (312) at a fourth point in time as a fourth position (303b),
wherein the control unit (40) determines a second distance vector between the first position (1220, 1240) and the fourth position (303b),
wherein the control unit (40) outputs an information (1250, 1260, 1450, 1460) based on the second distance vector together with an image (1200, 1300, 1400) captured at or after the fourth point in time.

8. The device according to one of the preceding claims, **characterized in that** the control unit (40) checks whether the determined first position (1220) is visible in a displayed image (1200, 1300, 1400),
wherein the endoscope (312) has captured the displayed image (1200, 1300, 1400) after the first point in time, and
wherein the control unit (40) inserts an optical marking at the first position (1220) into the displayed image (1200, 1300, 1400).

9. The device according to one of the preceding claims, **characterized in that** the control unit (40) inserts a vector arrow (1250, 1450) on a portion of a line between the center of the displayed image (1200, 1300, 1400) in the direction of the first position and inserts it into the displayed image (1200, 1300, 1400).

10. The device according to claim 9, **characterized in that** by a user input via a user interface (42) or automatically by the control unit (40) at least a second reference point is set, that the control unit (40), dependent on a preset selection parameter, optionally generates a first vector arrow (1250) and/or a second vector arrow (1450) and inserts it into the displayed image (1200, 1300, 1400).

11. The device according to one of the preceding claims, **characterized in that** the control unit (40) outputs an acoustic signal dependent on the value of the determined distance vector or a visual information dependent on the value of the determined distance vector or a haptic information for signaling the correct movement direction via a manual input device of a user interface (42).

12. The device according to one of the preceding claims, **characterized in that** the control unit (40) inserts the first reference point (1220) and possibly a further reference point as a respective reference point marking or as a marking of an object present at the reference point (1220) into a 3D model of a patient (18) to be operated.

13. The device according to one of the preceding claims, **characterized in that** the endoscope (312) is a stereo endoscope with a common optical axis (316), the control unit (40) determining the respective position as a position of a point of the common optical axis (316) of the stereo endoscope in the object plane (304b) or as a position of a point of the optical axis (316) of the stereo endoscope at a distance to the object plane (304b) or as a position of a line segment on the optical axis (316) of the stereo endoscope with a point (303b) in the object plane (304b), or
that the endoscope (312) is a stereo endoscope with two separate imaging optical systems, the optical axes of which are parallel, wherein the control unit (40) determines the respective position as a position of a point of an axis running between the optical axes of the stereo endoscope in the object plane (304b), or as a position of a point of an axis running between the optical axes of the stereo endoscope at a distance to the object plane (304b), or as a position of a line segment on the axis running between the optical axes of the stereo endoscope with a point (303b) in the object plane (304).

14. A system for robot-assisted surgery, in particular for a telerobot-assisted procedure, with a device (12) according to one of the preceding claims, wherein the system further comprises the user interface (42),
wherein the user interface (42) comprises at least an input device for the input of at least one input command,
wherein the at least one control unit (40) or a further control unit (40) controls actors of the system (10) for robot-assisted surgery such that the endoscope (312) connected to a manipulator arm (16b) and/or a surgical instrument (308, 310) for tissue manipulation connected to a further manipulator arm (16a) is positioned by at least one drive unit dependent on the input command.

## Revendications

1. Dispositif pour déterminer et recouvrir un point de référence pendant une intervention chirurgicale,
avec un endoscope (312), qui acquiert plusieurs images les unes après les autres en tant que séquence d'images et génère des données d'images correspondant aux images, avec au moins une unité de commande (40) et au moins une unité d'affichage (44),
dans lequel la au moins une unité de commande (40) traite les données d'images et sort les images (1200, 1300, 1400) correspondant aux données d'images sur la au moins une unité d'affichage (44),
dans lequel l'unité de commande (40) est conçue pour établir la position d'un point de l'axe optique de l'endoscope (312) ou d'un axe parallèle à l'axe optique (316) de l'endoscope (312) dans le plan d'objet (304b) ou la position d'un trajet sur l'axe optique (316) de l'endoscope (312) ou d'un axe parallèle à l'axe optique (316) de l'endoscope (312) avec un point (303b) dans le plan d'objet (304b) à un premier moment en tant que première position,
dans lequel la première position peut être déterminée en tant que premier point de référence (1220) par une entrée de commande utilisateur par l'intermédiaire d'une interface utilisateur,
dans lequel l'unité de commande (40) est conçue en outre pour établir la position d'un point de l'axe optique (316) de l'endoscope (312) ou de l'axe parallèle à l'axe optique (316) de l'endoscope (312) dans le plan d'objet (304b) ou la position du trajet sur l'axe optique (316) de l'endoscope (312) ou de l'axe parallèle à l'axe optique (316) de l'endoscope (312) à un deuxième moment en tant que deuxième position (303b),
dans lequel l'unité de commande (40) est conçue en outre pour établir un premier vecteur de distance entre la première position (1220) et la deuxième position (303b),
dans lequel l'unité de commande (40) sort sur l'unité d'affichage (44) une information (1450, 1560) se basant sur le premier vecteur de distance conjointement avec une image (1400) acquise au deuxième moment ou après celui-ci.

2. Dispositif selon la revendication 1, **caractérisé en ce que** l'unité de commande (40) acquiert une partie d'image de l'environnement de la première position établie lors de l'acquisition de la première position (1220) et, pour recouvrir la première position (1220) dans une autre image (1400) acquise après l'établissement de la première position (1220), la compare avec au moins une partie d'image de l'autre image.

3. Dispositif selon l'une quelconque des revendications précédentes, **caractérisé en ce que** l'unité de commande (40) établit la première position (1220, 1240) à partir d'une première image (1200, 1300, 1400) acquise à l'aide de l'endoscope (312) ou à partir de la position de l'endoscope (312) au premier moment, et
**que** l'unité de commande (40) établit la deuxième position (303b) à partir d'une deuxième image (1200, 1300, 1400) acquise après la première image (1200, 1300, 1400) à l'aide de l'endoscope (312) ou à partir de la position de l'endoscope (312) au deuxième moment.

4. Dispositif selon l'une quelconque des revendications précédentes, **caractérisé en ce que** l'unité de commande (40) établit les positions respectivement à l'aide de la chaîne cinématique d'un bras manipulateur (16b), relié à l'endoscope (312), d'un système de robot chirurgical (10).

5. Dispositif selon l'une quelconque des revendications précédentes, **caractérisé en ce que** l'endoscope (312) acquiert au premier moment une première image (1200, 1300, 1400), et que l'endoscope (312) acquiert au deuxième moment une deuxième image (1200, 1300, 1400), ou
**que** l'endoscope (312), avant ou lors de l'établissement de la première position, acquiert une première image (1200, 1300, 1400) et que l'endoscope (312), avant ou lors de l'établissement de la deuxième position, acquiert une deuxième image (1200, 1300, 1400).

6. Dispositif selon l'une quelconque des revendications précédentes, **caractérisé en ce que** l'endoscope (312) acquiert et sort, après la deuxième image (1200, 1300, 1400), à un troisième moment, au moins une autre, troisième, image (1200, 1300, 1400), dans lequel la position, l'orientation, la rotation, la situation et/ou l'agrandissement de l'endoscope (312) entre l'enregistrement de la deuxième image (1200, 1300, 1400) et de la troisième image (1200, 1300, 1400) sont inchangés,
dans lequel l'unité de commande (40) sort l'information se basant sur le premier vecteur de distance conjointement avec la troisième image (1200, 1300, 1400).

7. Dispositif selon l'une quelconque des revendications précédentes, **caractérisé en ce que** l'endoscope (312) acquiert et sort, après la deuxième image (1200, 1300, 1400), au moins une autre, quatrième, image (1200, 1300, 1400) à un quatrième moment, et
dans lequel la position, l'orientation, la rotation, la situation et/ou l'agrandissement de l'endoscope (312) entre le deuxième moment et le quatrième moment sont inchangés,
dans lequel l'unité de commande (40) établit la position d'un point (303b) de l'axe optique (316) de l'endoscope (312) ou de l'axe parallèle à l'axe optique (316) de l'endoscope (312) dans le plan d'objet (304b) ou la position d'un point de l'axe optique (316) de l'endoscope (312) ou de l'axe parallèle à l'axe optique (316) de l'endoscope (312) à distance du plan d'objet (304b) ou la position du trajet sur l'axe optique (316) de l'endoscope (312) ou de l'axe parallèle à l'axe optique (316) de l'endoscope (312) à un quatrième moment en tant que quatrième position (303b),
dans lequel l'unité de commande (40) établit un deuxième vecteur de distance entre la première position (1220, 1240) et la quatrième position (303b),
dans lequel l'unité de commande (40) sort une information (1250, 1260, 1450, 1460) se basant sur le deuxième vecteur de distance conjointement avec une image (1200, 1300, 1400) acquise au quatrième moment ou après celui-ci.

8. Dispositif selon l'une quelconque des revendications précédentes, **caractérisé en ce que** l'unité de commande (40) vérifie si la première position (1220) établie est visible dans une image (1200, 1300, 1400) affichée,
dans lequel l'endoscope (312) a acquis l'image (1200, 1300, 1400) affichée après le premier moment, et
dans lequel l'unité de commande (40) insère un marquage optique dans la première position (1220) dans l'image (1200, 1300, 1400) affichée.

9. Dispositif selon l'une quelconque des revendications précédentes, **caractérisé en ce que** l'unité de commande (40) génère un vecteur (1250, 1450) sur une partie d'une ligne entre le centre de l'image (1200, 1300, 1400) affichée en direction de la première position et l'insère dans l'image (1200, 1300, 1400) affichée.

10. Dispositif selon la revendication 9, **caractérisé en ce qu'**au moins un deuxième point de référence est établi par une entrée d'utilisateur par l'intermédiaire d'une interface utilisateur (42) ou de manière automatique par l'unité de commande (40), que l'unité de commande (40) génère sélectivement un premier vecteur (1250) et/ou un deuxième vecteur (1450) en fonction d'un paramètre de sélection préréglé et l'insère dans l'image (1200, 1300, 1400) affichée.

11. Dispositif selon l'une quelconque des revendications précédentes, **caractérisé en ce que** l'unité de commande (40) sort un signal acoustique dépendant de la valeur du vecteur de distance établi ou une information visuelle dépendant de la valeur du vecteur de distance établi ou une information haptique pour la signalisation de la direction de mouvement correcte par l'intermédiaire d'un dispositif manuel d'entrée d'une interface utilisateur (42).

12. Dispositif selon l'une quelconque des revendications précédentes, **caractérisé en ce que** l'unité de commande (40) insère le premier point de référence (1220) et éventuellement un autre point de référence respectivement en tant que marquage de point de référence ou en tant que marquage d'un objet se trouvant au point de référence (1220) dans un modèle 3D d'un patient (18) à opérer.

13. Dispositif selon l'une quelconque des revendications précédentes, **caractérisé en ce que** l'endoscope (312) est un endoscope stéréoscopique avec un axe optique (316) commun, dans lequel l'unité de commande (40) établit la position respective en tant que position d'un point de l'axe optique (316) commun de l'endoscope stéréoscopique dans le plan d'objet (304b) ou en tant que position d'un point de l'axe optique (316) de l'endoscope stéréoscopique à une distance du plan d'objet (304b) ou en tant que position d'un trajet sur l'axe optique (316) de l'endoscope stéréoscopique avec un point (303b) dans le plan d'objet (304b), ou
**que** l'endoscope (312) est un endoscope stéréoscopique avec deux optiques de reproduction séparées, dont les axes optiques sont parallèles, dans lequel l'unité de commande (40) établit la position respective en tant que position d'un point d'un axe s'étendant entre les axes optiques de l'endoscope stéréoscopique dans le plan d'objet (304b) ou en tant que position d'un point d'un axe s'étendant entre les axes optiques de l'endoscope stéréoscopique à une distance du plan d'objet (304b) ou en tant que position d'un trajet sur l'axe s'étendant entre les axes optiques de l'endoscope stéréoscopique avec un point (303b) dans le plan d'objet (304b).

14. Système pour la chirurgie robotisée, en particulier pour une procédure télérobotisée, avec un dispositif (12) selon l'une quelconque des revendications précédentes, dans lequel le système comprend en outre l'interface utilisateur (42),
dans lequel l'interface utilisateur (42) comprend au moins un dispositif d'entrée pour l'entrée d'au moins une commande d'entrée,
dans lequel l'au moins une unité de commande (40) ou une autre unité de commande (40) commande des actionneurs du système (10) pour la chirurgie robotisée, de telle sorte que l'endoscope (312) relié à un bras de manipulateur (16b) et/ou un instrument chirurgical (308, 310) relié à un autre bras de manipulateur (16a) pour la manipulation des tissus est positionné en fonction de la commande d'entrée à l'aide d'au moins une unité d'entraînement.
